(19) **Europäisches Patentamt**
**European Patent Office**
**Office européen des brevets**

(11) **EP 4 047 063 A1**

(12) **EUROPEAN PATENT APPLICATION**

(43) Date of publication:
**24.08.2022 Bulletin 2022/34**

(21) Application number: **22168879.9**

(22) Date of filing: **13.03.2017**

(51) International Patent Classification (IPC):
**C09D 167/08** *(2006.01)*     **C09D 5/16** *(2006.01)*
**C09D 9/00** *(2006.01)*     C02F 1/50 *(2006.01)*
A61L 2/232 *(2006.01)*     D21H 21/36 *(2006.01)*

(52) Cooperative Patent Classification (CPC):
(C-Sets available)
**A61L 2/232; C02F 1/50; C09D 5/1662;**
**C09D 5/1681; C09D 167/08; D21H 21/36;**
C02F 2303/20; C02F 2307/14     (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **11.03.2016 EP 16159994**

(62) Document number(s) of the earlier application(s) in
accordance with Art. 76 EPC:
**20157827.5 / 3 708 623**
**17709998.3 / 3 426 739**

(71) Applicant: **Dr. Klaus Schepers Patentverwaltung**
**35619 Braunfels (DE)**

(72) Inventors:
• **SCHEPERS, Klaus**
**35619 Braunfels (DE)**

• **MISCHO, Horst**
**54295 Trier (DE)**
• **BIRKEL, Alexander**
**64287 Darmstadt (DE)**
• **SABANOV, Elena**
**33129 Delbrueck (DE)**
• **BREMSER, Wolfgang**
**33102 Paderborn (DE)**

(74) Representative: **Lederer & Keller Patentanwälte**
**Partnerschaft mbB**
**Unsöldstraße 2**
**80538 München (DE)**

Remarks:
This application was filed on 19-04-2022 as a
divisional application to the application mentioned
under INID code 62.

(54) **USE OF ALKYD RESINS**

(57) The present invention relates to the use of alkyd resins for disinfecting purposes. It further relates to the use of alkyd resins for disinfecting purposes in antimicrobial surfaces, paints or coatings.

EP 4 047 063 A1

(52) Cooperative Patent Classification (CPC): (Cont.)

C-Sets
**C09D 167/08, C08L 61/28**

**Description**

The present invention relates to the use of alkyd resins for disinfecting purposes. It further relates to the use of alkyd resins for disinfecting purposes in antimicrobial surfaces, paints or coatings.

In many industrial and domestic processes waste water is produced or fresh water is to be stored for later purposes. As essentially all water sources provide non-sterile water, in fresh water and in particular in waste water there is the risk of microbiological contamination during storage or processing.

Many ways are known in the art to avoid creation of microbiological contaminations, in particular biofilms, in containers for the storage of drinking water, waste water, surface water or similar liquids. Further, several methods are known to avoid microbiological contamination of surfaces, e.g. in hospitals or slaughterhouses. For example, usually the surfaces of containers are cleaned by heat treatment, e.g. by rinsing the surfaces or containers with hot liquids or gases, e.g. hot water or steam. Further, treatment by ozone or chlorine containing gases is known for disinfecting surfaces and containers and cleaning and removal of organic contaminations.

It is also known to apply ultraviolet (UV-) radiation to disinfect surfaces, containers, and even liquids. It is also known to add certain substances to the liquids, such as titanium dioxide, or to apply titanium dioxide to a surface for disinfecting or cleaning purposes. However, these surfaces and substances require UV-radiation for activating the disinfecting efficacy of titanium dioxide.

Further, it is known to apply silver ions or copper ions to solutions in order to reduce the microbiological contamination.

All these measures and procedures are disadvantageous as they waste a lot of energy, require high apparatus cost and costs of operation in order to be sufficiently effective, or rather expensive substances, such as silver ions or copper ions, have to be used, the latter having further disadvantages with regard to water and soil pollution. Further, using substances which are dangerous to handle, e.g. ozone or chlorine containing substances, as well as ultraviolet radiation, is disadvantageous and requires additional safety measures.

The use of alkyd resins is known in the art for several purposes, e.g. in synthetic coating and paint industry. However, there was the need, in order to achieve antimicrobial effects in e.g. paints, to add specific antimicrobial agents.

For example US 4,039,494 describes a paint coating to prevent fungal or microbial growths using alkyd resins with an equivalent amount of organotin compound, so that the organotin is chemically incorporated into the resin.

WO 2010/04903 A1 discloses a wood impregnation method, in which an emulsion is absorbed into wood by means of pressure difference. The emulsion contains a reaction product of a fatty acid obtained from a natural oil and a polyalcohol, an emulgator and water. In this way the dimension stability and the water repulsion of wood can be physically improved. It is furthermore said that in addition a good biological durability is achieved. In the examples the resin is prepared in the presence of gum rosin, which is known for its antimicrobial activity. Thus, the emulsion contains a biocidal compound as an internal biocide within the resin. Additionally, the reaction product of a fatty acid with a polyalcohol does not result in an alkyd resin.

There is still a need for further compounds, which exhibit antimicrobial and in particular antibacterial activity and which preferably can provide antimicrobial and in particular antibacterial properties to at least part of a surface of a substrate.

It has been surprisingly found that alkyd resins alone without any additional external or internal biocides provide excellent antimicrobial efficiency when applied to surfaces, which are in contact with liquids, in particular with waste water, and thereby avoid microbiological contamination of surfaces and of the liquid and lead to a reduction of biofilm formation.

Therefore, the present invention relates to the use of an alkyd resin for providing antimicrobial properties to at least part of a surface of a substrate.

Applicants do not wish to be bound to any theory, but it is believed that alkyd resins by themselves exhibit an antimicrobial and in particular antibacterial effect due to physical interaction between the hydrophobic fatty acid side chains of the alkyd polymer and the cell membranes of for example bacteria contacting a surface equipped with the alkyd resin. The antimicrobial efficiency of alkyd resins therefore is based on mere physical or mechanical action.

The alkyd resins according to the present invention provide these effects in a liquid form as well as after they have been cured.

Said purposes can be characterized by the ability to deter, render harmless, destroy and/or exert a controlling effect on all forms and/or parts of microbial life (e.g. bacteria, viruses, fungi, spore forms, etc.); and/or by the ability to remove and/or reduce offensive and/or unpleasant scents.

The term "antimicrobial", as used herein, refers to the ability of the alkyd resin to kill microorganisms.

The term "microorganism", as referred to herein, includes bacteria, viruses, fungi and algae. Preferably, the term "microorganism", as used herein, refers to bacteria.

The ability to kill microorganisms results in disinfecting, antifouling, self-sanitizing and and/or biocidal effects. In this context "antifouling" refers to the ability to prevent or remove biofouling on wetted surfaces. Biofouling is the accumulation of microorganisms, plants, algae or animals on wetted surfaces and killing such microorganisms, including algae, supports the prevention or removal of biofouling.

The term "substrate", as referred to herein, includes bodies of any material, including for example polymers, metal, glass and stone, as well as coatings on such bodies. The bodies may have any shape including for example plates, sheets, fibers and tubes.

The term "alkyd resin", as used herein, refers to a polyester modified by the addition of fatty acids and/or other components. Alkyd resins are derived from a di-, tri- or polyol and a di-, tri- or polycarboxylic acid or carboxylic acid anhydride. The monomers as such are not encompassed by the term "alkyd resin".

The di-, tri- or polyacid can for example be selected from the group consisting of oxalic acid, malonic acid, succinic acid, glutaric acid, adipic acid, pimelic acid, suberic acid, azelaic acid, cebacic acid, maleic acid, fumaric acid, glutaconic acid, malic acid, aspartic acid, glutamic acid, tartaric acid, phthalic acid, isophthalic acid, terephthalic acid and/or mixtures thereof. Anhydrides of these acids are also suitable. Preferably, the acid residue in the polyester is a diacid residue.

The di-, tri- or polyalcohol can for example be selected from the group consisting of ethylene glycol, butylene glycol, pentanediol, neopentyl glycol, hexylene glycol, diethylene glycol, dipropylene glycol, triethylene glycol, glycerol, trimethylolethane, trimethylopropane, pentaerythritol, methylglucoside, sugars, sugar alcohols, such as mannitol, xylitol and sorbitol and/or mixtures thereof.

The polyester backbone in the alkyd resin can be modified by any organic residue, preferably a hydrophobic residue, more preferably a hydrophobic residue which does not contain any additional reactive groups as defined below. Preferably, the polyester is modified with an acid residue, more preferably with a fatty acid residue.

The term "fatty acid" refers to a carboxylic acid having an aliphatic chain. In one embodiment, the aliphatic chain is saturated. In another embodiment, the aliphatic chain is unsaturated. The number of C-atoms in the aliphatic chain can be in the range from 1 to 40 C-atoms, preferably 1 to 26 C-atoms or 1 to 18 C-atoms. Even more preferably the number of C-atoms in the aliphatic chain is at least 6, such as at least 10 or at least 15. More preferably the number of C-atoms in the aliphatic chain is in the range of 6 to 40, 6 to 26, 6 to 20, 10 to 40, 10 to 26, 10 to 20, 15 to 40, 15 to 26 or 15 to 20; 15 to 20 being particularly preferred.

Preferably, the fatty acid is selected from the group consisting of caproic acid, caprylic acid, capric acid, myristoleic acid, palmitoleic acid, sapienic acid, oleic acid, elaidic acid, vaccenic acid, linoleic acid, linoelaidic acid, $\alpha$-hnolenic acid, arachidonic acid, palmitic acid, stearic acid, lauric acid, myristic acid and/or combinations thereof. In another embodiment, the fatty acid can be di- or trifunctional and is selected from the list consisting of adipic acid, fumaric acid, isophthalic acid, maleic acid, phthalic acid, succinic acid, citric acid and/or combinations thereof. In a further embodiment, the fatty acid is an anhydride of the above named fatty acids.

In one embodiment, the alkyd resin is a linear polyester. A linear polyester, as defined herein is a difunctional molecule and has two reactive sites.

In another embodiment, the alkyd resin is a branched polyester containing fatty-acid side groups. A branched polyester, as defined herein is a tri-, tetra-, or higher functional molecule and has three, four, or more reactive sites. Branched polyester may form, as linear polyester, molecular chains, but, in addition, branching connections are formed, which result in a three-dimensional network (cross-linking).

In one embodiment, the alkyd resin is further modified with an unsaturated or saturated oil or the alkyl resin contains such oil.

Preferably, the unsaturated or saturated oil is selected from the group consisting of tung oil, linseed oil, sunflower oil, safflower oil, walnut oil, soybean oil, fish oil, corn oil, tall oil, dehydrated castor oil, coconut oil, rapeseed oil, peppermint oil, lavender oil, safflower oil, walnut oil, fish oil, corn oil, tall oil, dehydrated castor oil, cumin oil, flax oil, vernonia oil and/or mixtures thereof. Linseed oil, sunflower oil, coconut oil, rapeseed oil, peppermint oil, lavender oil and soybean oil, safflower oil, walnut oil, fish oil, corn oil, tall oil, dehydrated castor oil, cumin oil, flax oil and vernonia oil being particularly preferred.

The above oils can be present in the alkyd resin as such or as derivatives thereof, for example as their fatty acid, monoester, fatty acid diester, and esters with other carboxylic acids, such as ascorbic acid, acetic acid, propionic acid, benzoic acid or lactic acid.

Further possible additives in the alkyd resin are for example fatty acids, in particular those as described above, oct-1-en-3-ol, citronellal and pheromones of clothing mots, such as for example (Z, E)-tetradeca-9,12-dienyl acetate.

Alkyd resins comprising an unsaturated or saturated oil are classified by the amount of oil based on the total weight of the resin. In one embodiment, the amount of unsaturated or saturated oil is higher than 60 wt.-% (long-oil alkyd resin), between 40 wt.-% and 60 wt.-% (medium-oil alkyd resin), or lower than 40 wt.-% (short-oil alkyd resin) based on the total weight of the alkyd resin. In a preferred embodiment the alkyd resin is a medium-oil alkyd resin or a long-oil alkyd resin.

In one embodiment the alkyd resin is WorléeKyd B 845 (Worlée-Chemie GmbH, Germany), Setal 196 XX (Nuplex, USA, Kentucky) or Alkydal F26 XX, Alkydal F251 X (Covestro, Germany).

In one embodiment, the alkyd resin additionally reduces the growth of a biofilm on the surface of the substrate. The term "additionally" as used herein has the meaning of "simultaneously" and/or "sequentially".

The term "biofilm", as used herein, refers to an assembly of microorganisms wherein cells stick to each other on the surface of a substrate. The term "growth of biofilm" or "biofilm growth", as used herein, refers to the microorganism built-up adhering to a surface of a substrate.

The term "reduction of the growth of a biofilm", as used herein, refers to the decrease, preferably the inhibition, of the microorganism built-up on a long term scale, i.e. at least 1 week, preferably at least 1 month, more preferably at least 1 year, even more preferably at least 3 years, on the surface of a substrate comprising an alkyd resin as described herein compared to the surface of a substrate not comprising an alkyd resin as described herein.

In one embodiment, the alkyd resins as described herein are particularly suitable for simultaneously imparting antimicrobial properties to a surface of a substrate and reducing, particularly inhibiting, the growth of a biofilm on the surface of the substrate, thereby reducing, preferably inhibiting, the growth of biofilm on a substrate surface and maintaining the microbial efficacy of the alkyd resins on a long term time scale (at least 2 weeks, preferably at least 1 month, more preferably at least 1 year, even more preferably at least 3 years).

In one embodiment, the alkyd resin is a drying or a non-drying alkyd resin.

Drying alkyd resins cure at approximately 20°C or by drying at 60°-80°C. Non-drying alkyd resins do not dry at these temperatures, but are cured by baking.

In one embodiment, the alkyd resin further comprises a siccative (oil-drying agent), which catalyzes the curing of the alkyd resins. Siccatives are typically derived from cobalt, manganese and iron ions.

In another embodiment, the alkyd resin does not contain any siccatives.

A particular advantage of the use, according to the present invention, is the finding that alkyd resins itself without the requirement of any further biocidally acting compound provides the desired antimicrobial effect. Therefore, in a particularly preferred embodiment of the present invention the alkyd resin is free of any additional antimicrobial compound, in particular free of any biocidal product as defined in Article 3(1)(a) of the Regulation (EU) No. 528-2012 of the European Parliament and the Counsel of 22 May 2012. In particular, the alky resin used in the present invention does not contain any nanoparticles, such as metal nanoparticles, in particular silver nanoparticles. Preferably the alkyd resin is free of silver, organotin and gum rosin.

In further embodiment the alkyd resin used according to the present invention does not contain any internal biocides, preferably the alkyd resin does not contain any internal biocides and any external biocides. In this context "internal biocides" are understood as compounds having biocidal activity (in particular those compounds as defined in Article 3(1)(a) of the Regulation (EU) No. 528-2012 of the European Parliament and the Counsel of 22 May 2012), which are chemically bound to the alkyd resin, such as the gum rosin in the alkyd resin of example 1 of WO 2010/040903 or the organotin compound in the alkyd resin of US 4,039,494. "External biocides" are to be understood as those biocidal compounds which are present in the alkyd resin, but which are not chemically bound to the alkyd polymer.

Since it is believed that the antimicrobial effect of the alkyd resin is due to a mechanical interaction between the hydrophobe fatty acid residue side chains of the polyester polymer and the cell membranes of for example bacteria, it is desirable that the fatty acid residues have a certain hydrophobicity but do not react with the cell membranes. It is therefore preferred that the fatty acid residues in the alkyd resins do not contain any reactive functional groups, such as hydroxy, amino, sulfo, phosphato, carboxy, carboxy-amid, cyano, etc. Possible unsaturations in the fatty acid residues are not considered as reactive functional groups in the sense of the present invention.

The above described qualities can be used to modify surfaces of substrates. Wherein one or more parts (e.g. materials, additives, surface layers, inner layers, exterior, interior, etc.) of the said substrate comprise an alkyd resin as described herein, and/or one or more of the surfaces (e.g. functional surfaces) of the said substrate are at least partially coated with surface layers, paints or coatings, wherein said surface layers, paints or coatings comprise an alkyd resin.

The substrates can be selected from the group consisting of: a polymer body, a paint, a varnish, a coating, an ink, glass fibers and an inorganic oxide material, containers for storage of drinking water (e.g. in the beverage industry), waste water, or surface water; containers for waste decomposition; containers for water purification; hospitals, medical equipment and devices, wound dressings, diapers, household appliances, water boilers, heating systems, slaughterhouses, ships, boats, roof coverings, roof tiles, indoor tiles, outdoor tiles, kitchen rooms, sinks, bathroom equipment, wash closets, toilets, portable toilets, ceramics, polymers, fibers, rain water sewers, exterior façades, elements of façades, pools, pumps, tubing, walls, floorings, laminates, technical textiles, activewear fabrics, textile fibres, paper, wood, apparatus for air and water purification, apparatus for soil decontamination, window glass (e.g. self-cleaning windows), mirrors (e.g. anti-fog coatings for mirrors and/or glass), filter materials such as nonwovens, respirator masks, air filters, such as air-conditioner filters, water filters, and activated-carbon filters for water, air purification, and/or (food) packaging.

In one embodiment of the present invention the alkyd resin is further combined with a polymer such as a thermoplastic polymer, an elastomer, a thermoplastic elastomer, a duroplast or a mixture thereof.

The term "thermoplastic", as used herein, refers to a polymer that becomes pliable or moldable above a specific temperature and solidifies upon cooling. Examples for thermoplastic polymers include but are not limited to polyacrylates, acrylonitrile-butadiene-styrenes, polyamides such as nylon, polyacetic acid, polybenzimidazole, polycarbonate, polyether sulfone, polyetherether ketone, polyetherimide, polyethylene, polyphenylene oxide, polyphenylen sulfide, polypropylene, polystyrene, polyvinylchloriode, polyethyleneterephthalate, polyurethane, polyester and polytetrafluoroethylene (e.g. Teflon).

The term "elastomer", as used herein, refers to a polymer with viscoelasticity (having both viscosity and elasticity). Examples for elastomers include but are not limited to unsaturated rubbers such as natural polyisoprene (natural rubber), synthetic polyisoprene, polybutadiene, chloroprene rubber, butyl rubber, styrene-butadiene rubber, (hydrogenated) nitrile rubber, saturated rubbers such as ethylene propylene rubber, ethylene propylene diene rubber, epichlorohydrin rubber, polyacrylic rubber, silicone, silicone rubber, fluorosilicone rubber, fluoro- and perfluoroelastomers, and ethylene-vinyl acetate.

The term "thermoplastic elastomer", as used herein, refers to a class of copolymers or a physical mix of polymers which consists of materials with both thermoplastic and elastomeric properties. Examples for thermoplastic elastomers include but are not limited to styrenic block copolymers, polyolefin blends, elastomeric alloys, thermoplastic polyurethanes, thermoplastic copolyester, and thermoplastic polyamides.

The term "duroplast", as used herein, refers to a polymer which is no longer pliable after curing. Examples for duroplasts include but are not limited to aminoplasts, phenoplasts, epoxy resins, polyacrylates, polyurethanes, polyesters, urea formaldehyde resins, melamine formaldehyde resins, and phenol formaldehyde resins.

In a more preferred embodiment of the present invention, the substrate comprises a polymer selected from the group consisting of polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof.

Further examples of polymers are polyolefins, such as polyethylene and polypropylene, or melamine resins (melamine-formaldehyde resins). Melamine-formaldehyde resins form via the condensation of formaldehyde with melamine. For example, the amount of the alkyd resin is in the range of 10 to 90 wt.-% and the amount of the melamine resin is in the range of 10 to 90 wt.-% based on the total weight of the two resins. In a preferred embodiment, the alkyd resin is in the range of 50 to 90 wt.-% and the amount of the melamine resin is in the range of 10 to 50 wt.-% based on the total weight of the two resins. Particularly preferred, the alkyd resin is about 70 wt.-% and the amount of the melamine resin is about 30 wt.-% based on the total weight of the two resins. An example of a suitable melamine resin is Maprenal MF 900 (INEOS Melamines GmbH, Germany). It was found that the combination of an alkyd resin with a melamine resin improves surface hardness and scratch resistance and reduces curing time.

The incorporation of the alkyd resin into a polymer body can e.g. be achieved by mixing the alkyd resin with the polymer in order to obtain a dispersion or a compound of alkyd resin and polymer. Said dispersion or compound can then e.g. be submitted to injection molding or extrusion for forming the polymer body.

Alternatively, the single components of the alkyd resin can be mixed with the polymers before polymerization of the alkyd resin is carried out.

The resulting polymer can e.g. be submitted to compounding processes, pressureless processing techniques (e.g. casting, dipping, coating, foaming) or compression molding, rolling and calendaring, extrusion, blow molding or injection molding processes or drawing, thermoforming or printing for forming the polymer body.

The polymer body as described herein can also be obtained by drylaid, airlaid, spunlaid/meltblown or wetlaid processes, in particular if the polymer body is for use as fiber material or non-woven material.

The abbreviation "wt.-%" or "w/w", as used herein, means "weight percentage" and refers to the weight amount of a compound in relation to the (total) weight of a composition of compounds or of a substrate if nothing else is explicitly stated or obvious under the circumstances.

**Examples**

The value of the antimicrobial activity (R-value) is calculated as follows:

$$R = \log (B/A) - \log (C/A)$$

| A | mean value of colony-forming unit (CFU) of untreated surfaces, directly after application (0 h) |
|---|---|
| B | mean value of colony-forming unit (CFU) of untreated surfaces, after incubation (24 h, 36 °C, 90% relative humidity (rH)) |
| C | mean value of mean colony-forming unit (CFU) of treated surfaces, after incubation (24 h, 36 °C, 90% relative humidity (rH)) |

**Example 1**

Alkyd resins and combinations of alkyd resin with melamine resins were tested on their antimicrobial effect.

The antimicrobial tests were performed on Polypropylen (PP) - plates having a size of 15 $cm^2$. The area of the blind value testing was 16 $cm^2$. Therefore, the values of the antimicrobial testing were extrapolated to 16 $cm^2$. The microorganism tested for was the gram-positive bacterium Staphylococcus aureus (DSM 799).

The results are summarized in the following table 1:

Table 1

| Sample | CFU / $cm^2$ [mean of three tests] | | R-value |
|---|---|---|---|
| | after 0 hours | after 24 hours | |
| blank value | $6.0 \times 10^7$ | $1.5 \times 10^8$ | - |
| WorleeKyd B 845 | --- | < 4 | > 7.66 |
| Alkydal F 26 X | --- | < 4-50 | > 7.14 - 5.87 |
| Alkydal F 251 X / Maprenal MF 900 (70 wt.-%/30 wt.-%) | --- | < 4 | > 6.87 |

It can be seen from the above table that all of the tested alkyd resins as well as the combination of alkyd resin with melamine resin show excellent antimicrobial effects.

**Example 2**

The test as described in example 1 was repeated. Prior to testing, the resins were washed with demineralized water or tap water over 24 hours.

The results are summarized in the following table 2:

Table 2

| Sample | CFU / $cm^2$ [mean of three tests] | | R-value |
|---|---|---|---|
| | after 0 hours | after 24 hours | |
| blank value | $1.6 \times 10^7$ | $4.5 \times 10^7$ | - |
| WorleeKyd B 845 washed with demineralized water | --- | < 5000 | > 3.95 |
| WorleeKyd B 845 washed with tap water | --- | < 5000 | > 3.95 |
| WorleeKyd B 845 w/o siccative washed with demineralized water | --- | < 5000 | > 3.95 |

(continued)

| Sample | CFU / cm² [mean of three tests] | | R-value |
|---|---|---|---|
| | after 0 hours | after 24 hours | |
| WorleeKyd B 845 w/o siccative washed with tap water | --- | < 5000 | > 3.95 |
| Setal 196 XX washed with demineralized water | --- | < 5000 | > 3.95 |
| Setal 196 XX washed with demineralized water | --- | < 5000 | > 3.95 |
| Demineralized water: <1 µS conductivity | | | |

It can be seen from the above table that all of the tested alkyd resins show excellent antimicrobial effects even after washing over 24 hours in demineralized or tap water.

**Example 3**

The test as described in example 1 was repeated. Prior to testing, the resins were eluted 3 weeks in 100 ml tap water.

The results are summarized in the following table 3:

Table 3

| Sample | CFU / cm² | | R-value |
|---|---|---|---|
| | after 0 hours | after 24 hours | |
| blank value | $5.4 \times 10^1$ | $1.1 \times 10^8$ | --- |
| WorleeKyd B 845 without siccative | --- | 680 | 5.19 |
| Setal 196 XX | --- | $2.8 \times 10^5$ | 2.58 |

It can be seen from the above table that all of the tested alkyd resins show excellent antimicrobial effects even after elution in water over 3 weeks.

**Example 4**

The test as described in example 1 was repeated, with the modification that the gram-negative bacterium Escherichia coli (DSM 1116) was used as a test microorganism.

The results are summarized in table 4 below:

Table 4

| Sample | CFU / cm² [mean of three tests] | | R-value |
|---|---|---|---|
| | after 0 hours | after 24 hours | |
| blank value | $2.4 \times 10^7$ | $2.2 \times 10^8$ | --- |
| Alkydal F 26 X | --- | < 3 | > 6.93 |
| WorleeKyd B 845 | --- | < 3 | > 6.93 |
| Setal 196 XX | --- | < 3 | > 6.93 |

As can be seen from the table above, also against the gram-negative Escherichia coli, the different alkyd resins display high antimicrobial effectiveness; comparable in extent to what was observed for the gram-positive Staphylococcus aureus.

**Example 5**

The test as described in example 1 was repeated, with the modification that the mold Asperigillus brasiliensis (DSM 1988) was used as a test microorganism.

The results are summarized in table 5 below:

Table 5

| Sample | CFU / cm$^2$ [mean of three tests] | | R-value |
| --- | --- | --- | --- |
| | after 0 hours | after 24 hours | |
| blank value | $2.8 \times 10^7$ | $2.6 \times 10^7$ | --- |
| Alkydal F 26 X | --- | $6.0 \times 10^7$ | 0.57 |
| WorleeKyd B 845 | --- | $1.9 \times 10^6$ | 2.08 |
| Setal 196 XX | --- | $3.0 \times 10^7$ | 0.87 |

As can be seen from the table above, also against the mold Asperigillus brasiliensis, the different alkyd resins display antimicrobial effectiveness.

**Example 6**

In order to exclude that the antimicrobial effect was based on acidic pH of the resins, the pH was measured prior and after washing with a few milliliters of demineralized water and tap water.

The results are summarized in the following table 6:

Table 6

| | | pH | | |
| --- | --- | --- | --- | --- |
| | water | 0 h | 24 h | 72 h |
| WorleeKyd B 845 | demineralized | 5.56 | 4.83 | 5.41 |
| | tap | 7.49 | 7.81 | 7.96 |
| WorleeKyd B 845 without siccative | demineralized | 5.56 | 4.84 | 5.48 |
| | tap | 7.49 | 7.88 | 7.79 |
| Setal 196 XX | demineralized | 5.56 | 5.24 | 5.37 |
| | tap | 7.49 | 8.33 | 8.20 |

It can be seen from the above table that the pH of the tested alkyd resins does not alter significantly and thus it can be followed that the antimicrobial effect of the alkyd resins is not based on acidic pH.

**Example 7**

The alkyd resins and combination of alkyd resin with melamine resin were tested for their mechanical properties.

The results are summarized in the following table 7:

Table 7

| | Cross hatch test according to DIN EN ISO 2409 [adhesion] | Pencil hardness [scratch resistance] | Indentation hardness [mechanical strength] |
| --- | --- | --- | --- |
| WorleeKyd B845 | 0/0 | 6B | 47.6 |
| Alkydal F 251 X / Maprenal MF 900 (70 wt.-%/30 wt.-%) | 0/1 | F | 83.3 |

It can be seen from the above table that all of the tested alkyd resins as well as the combination of alkyd resin with melamine resin show excellent mechanical properties. The combination of 70 wt.-% alkyd resin with 30 wt.-% melamine resin even further improved adhesion, scratch resistance and mechanical strength of the resin.

**Example 8**

Alkyd resins and combination of alkyd resin with melamine resins were tested for their effectiveness against biofilm formation. For that, the test samples, as described in example 1, were added to individual wells of a multi-well plate. Biofilm medium, inoculated with Staphylococcus aureus at $10^6$ CFU/ml, was added to individual wells. The samples were removed after different times, rinsed with sterile water and dried. The dried samples were examined using scanning electron microscopy.

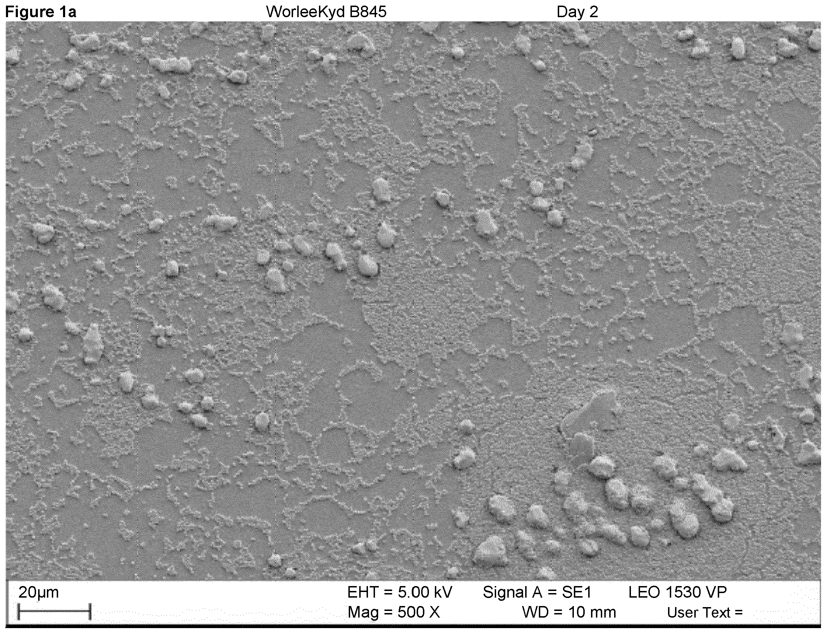

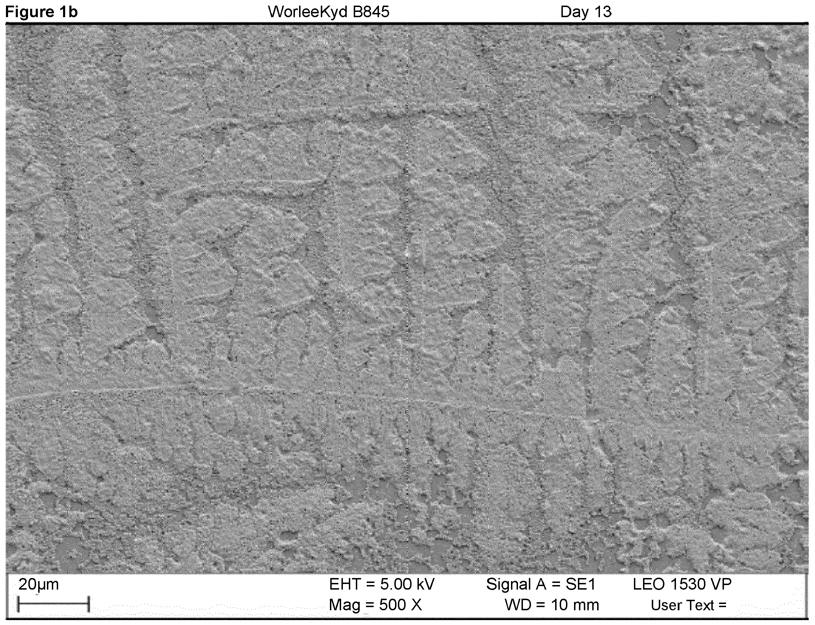

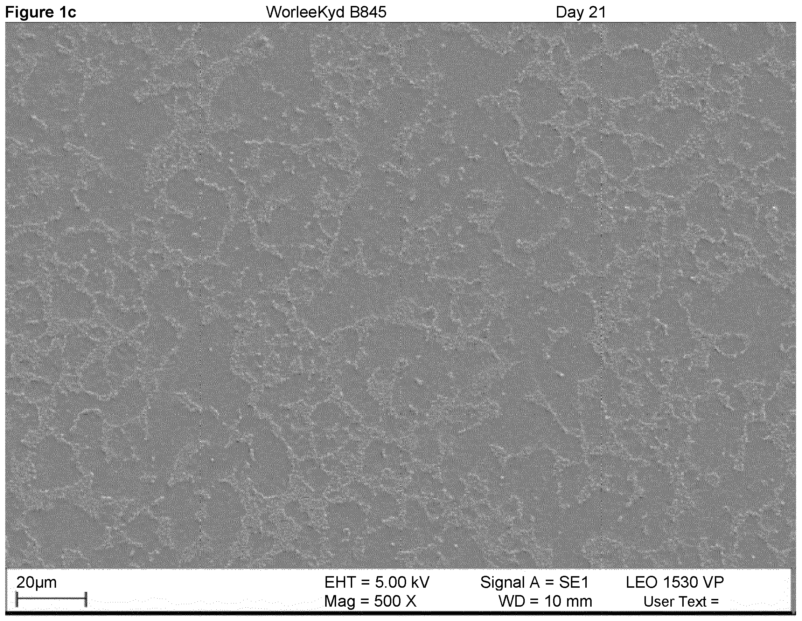

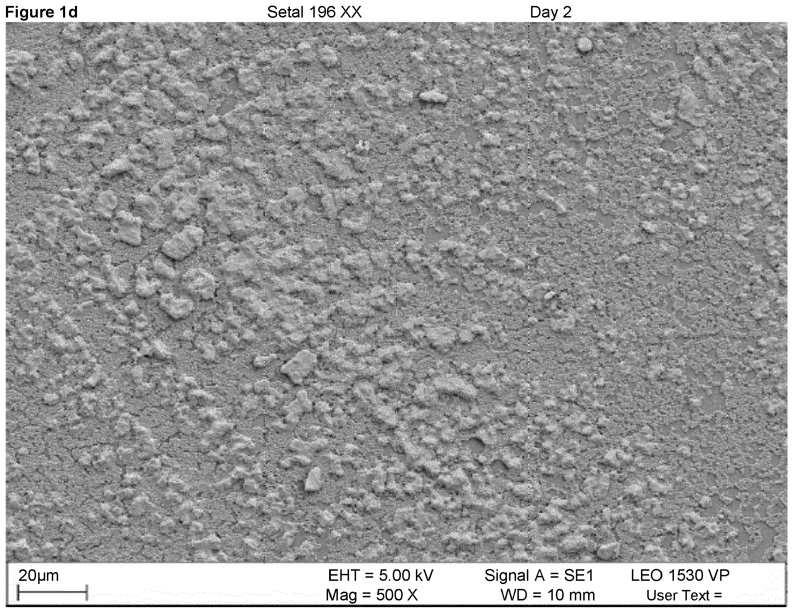

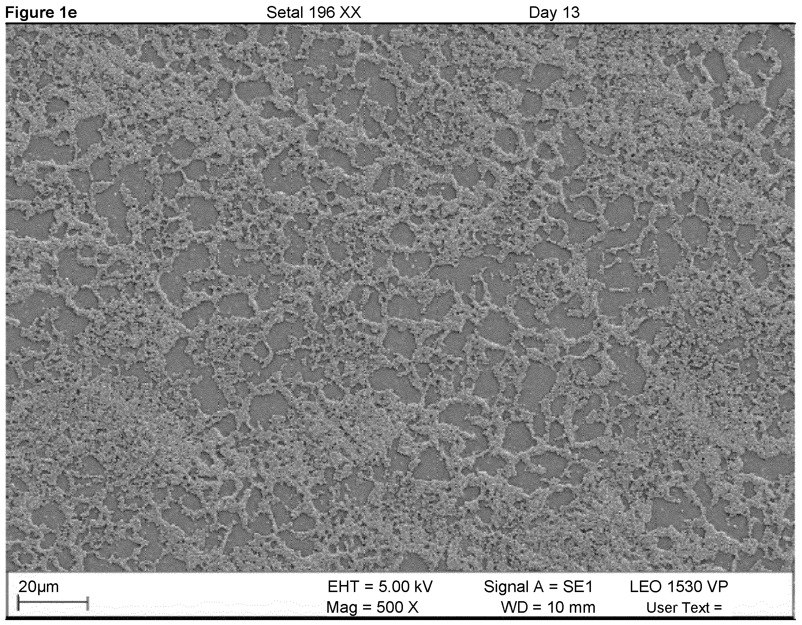

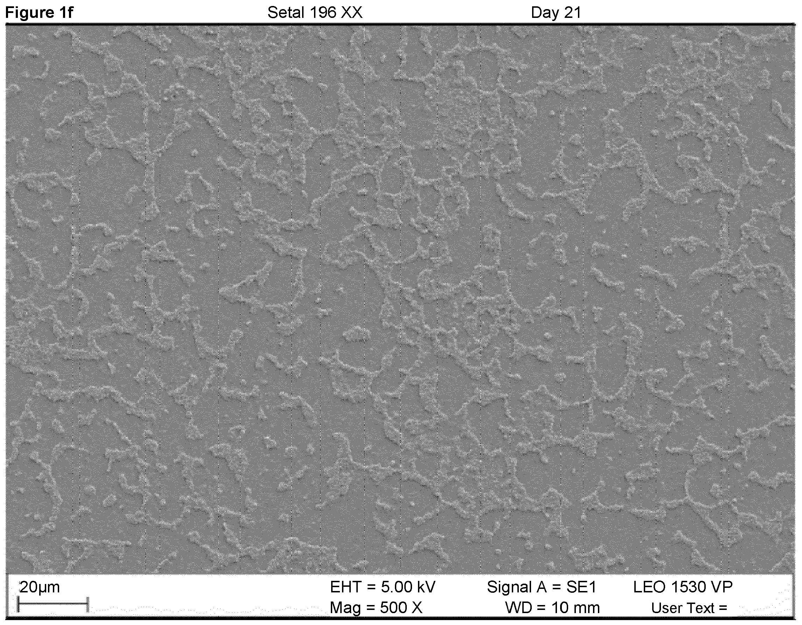

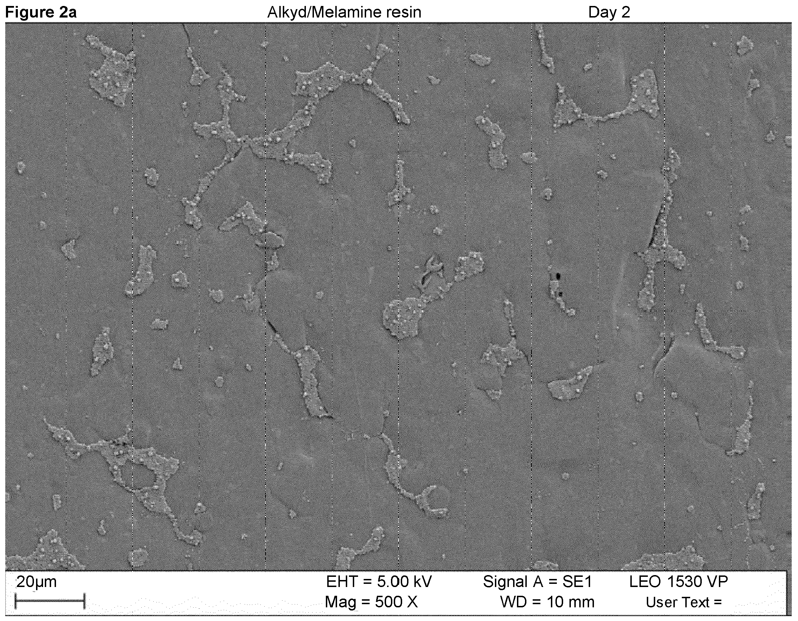

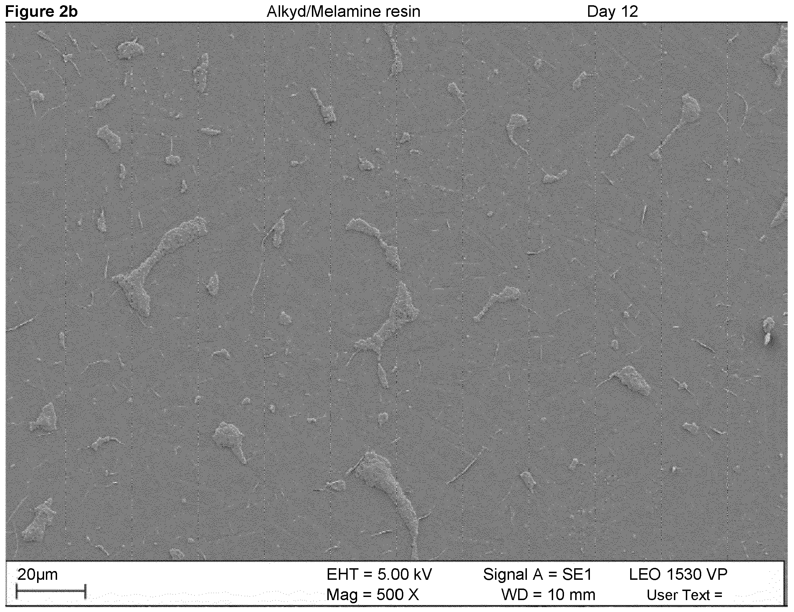

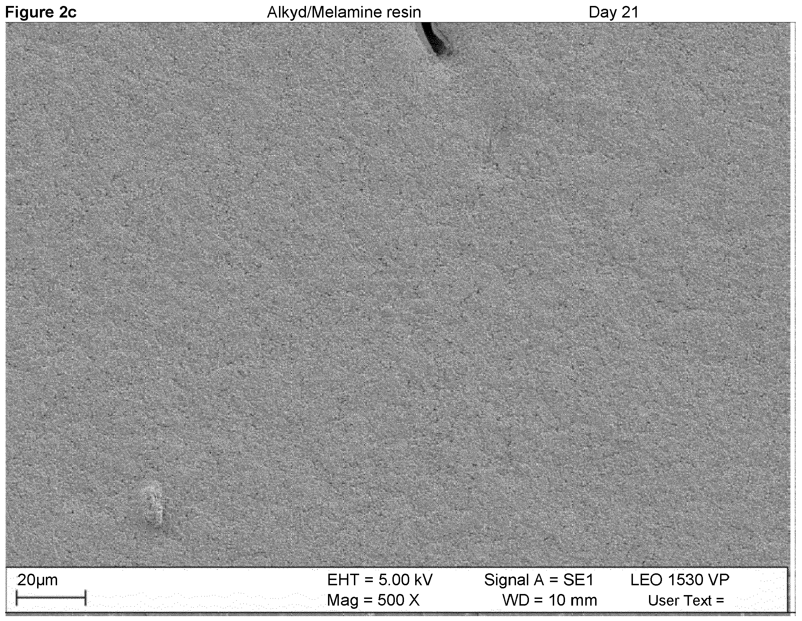

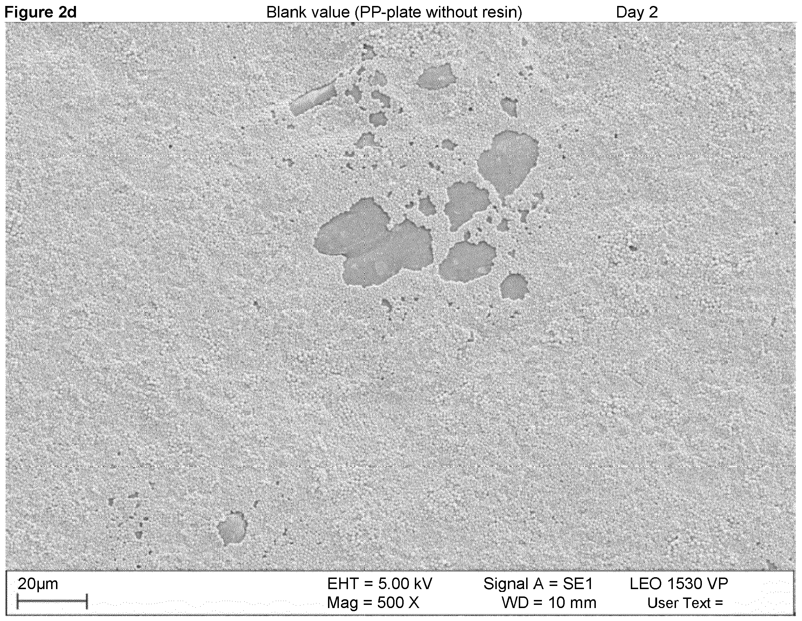

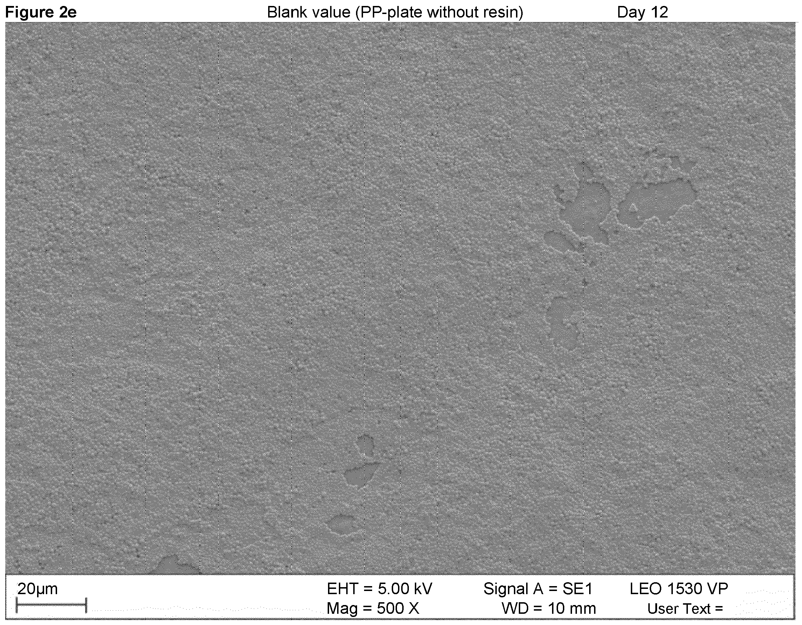

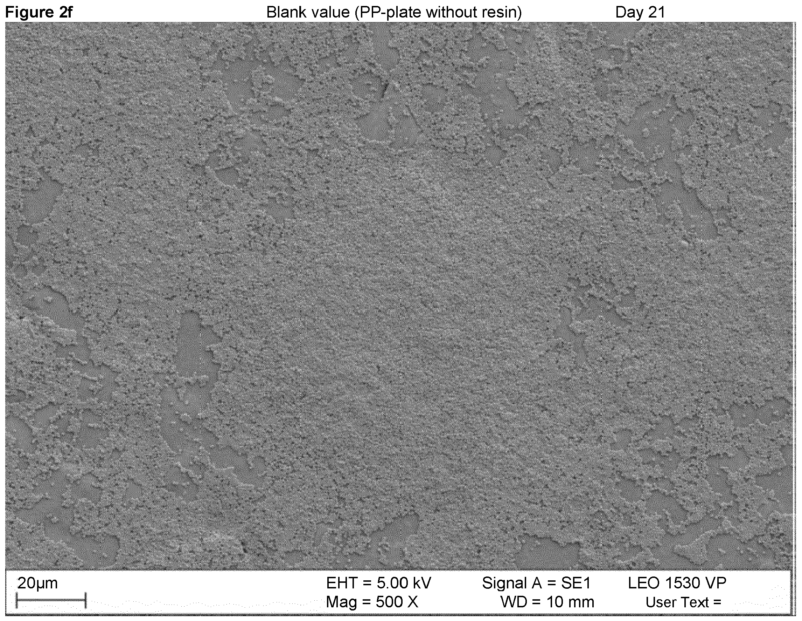

As can be seen from the images in Figures 1 and 2, for the alkyd resins after 21 days there are large gaps in the biofilm. For the alkyd resin with melamine resin, biofilm formation after 12 days is strongly reduced. This demonstrates that both alkyd resins and alkyd resins with melamine resins lead to a strong reduction in biofilm formation.

**Example 9**

The text as described in example 1 was repeated. Two alkyd resins obtained from Worlee-Chemie GmbH were tested, namely WorleeKyd B 845 and Worleesol E 330 (WSE 330). In order to exclude the presence of any low molecular weight compounds in the alkyd resin coatings, these were extracted for 24 hours in water, one hour in white spirit and one minute in butanol, respectively. For butanol the time of extraction was short because butanol is an excellent solvent also for the alkyd resin itself and it was found that the coating itself was destroyed after 2 minutes. Therefore an extraction time of only one minute was selected.

Extracting the alkyd resins with the solvents of different polarity ensures that the coating do not contain any small molecular weight residues which could influence the antimicrobial activity of the coating. The observed activities are therefore exclusively based on the alkyd resin itself.

The results are summarized in the following table 8:

Table 8

| Sample | CFU / cm$^2$ [mean of three tests] | | R-value |
| --- | --- | --- | --- |
| | after 0 hours | after 24 hours | |
| WorleeKyd B 845 + Siccative (blind) | --- | < 4 | > 7.15 |
| WorleeKyd B 845 + Sicc. + H$_2$O 24h | --- | < 4 | > 7.15 |
| WorleeKyd B 845 + Sicc. + white spirit 60 min | --- | < 4 | > 7.15 |
| WorleeKyd B 845 + Sicc. + butanol 1 min | --- | < 4 | > 7.15 |
| WSE 330 (blind) | --- | < 4 | > 7.15 |
| WSE 330 + H$_2$O 24h | --- | < 4 | > 7.15 |
| WSE 330 + white spirit 60 min. | --- | < 4 | > 7.15 |
| WSE 330 + butanol 1 min | --- | < 4 | > 7.15 |
| blank value | $1.5 \times 10^7$ | $4.8 \times 10^8$ | --- |

It can be seen from the data in the above table that those samples which were extracted with water, white spirit and butanol, respectively, do not exhibit a higher antimicrobial activity compared to the alkyd resin coating without extraction (blind). It is therefore evident that the observed antimicrobial activity is due to the alkyd resin as such and not due to the presence of any low molecular weight molecules.

**Example 10**

Two alkyd resins obtained from Worlee-Chemie GmbH were tested, namely WorleeKyd B 845 and Worleesol E 330 (WSE 330). After polymerization of the alkyd resins, the resins have been milled and used as an additive for polypropylene in an injection moulding process.

The results are summarized in the following table 9:

Table 9

| Sample | CFU / cm$^2$ [mean of three tests] | | R-value |
|---|---|---|---|
| | after 0 hours | after 24 hours | |
| WSE 330 before hardening (loading 8,4 % dry substance) in polypropylene | --- | $3.0 \times 10^5$ | 1.16 |
| WSE 330 after hardening and milling (loading 10%) in polypropylene | --- | $1.6 \times 10^5$ | 1.42 |
| WorleeKyd B 845 + Sicc. after hardening without milling (loading 10%) in polypropylene | --- | $4.0 \times 10^4$ | 2.03 |
| blank value | $2.4 \times 10^6$ | $4.3 \times 10^6$ | --- |

It can be seen from the data in the above table that alkyd resins can be incorporated in polyolefins as an additive by an injection moulding process in order to produce polyolefins with an antimicrobial functionality.

**Claims**

1. Use of an alkyd resin for providing antifungal properties to at least part of a surface of a substrate.

2. Use of an alkyd resin according to claim 1, wherein the alkyd resin is a polyester modified with fatty acids.

3. Use of an alkyd resin according to claim 1 or 2, wherein the alkyd resin is a linear polyester.

4. Use of an alkyd resin according to claim 1 or 2, wherein the alkyd resin is a branched polyester containing fatty-acid side groups.

5. Use of an alkyd resin according to any one of the preceding claims, wherein the alkyd resin is further modified with or contains an unsaturated or saturated oil.

6. Use of an alkyd resin according to claim 5, wherein the unsaturated or saturated oil is selected from the group consisting of linseed oil, sunflower oil, coconut oil, rapeseed oil, peppermint oil, lavender oil, soybean oil, safflower oil, walnut oil, fish oil, corn oil, tall oil, dehydrated castor oil, cumin oil, flax oil, wood oil, vernonia oil and/or mixtures thereof.

7. Use of an alkyd resin according to claim 5 or 6, wherein the amount of unsaturated or saturated oil is higher than 60 wt.-% (long-oil alkyd resin), between 40 wt.-% and 60 wt.-% (medium-oil alkyd resin), or lower than 40 wt.-% (short-oil alkyd resin) based on the total weight of the alkyd resin.

8. Use of an alkyd resin according to claim 7, wherein the alkyd resin is a medium-oil alkyd resin or a long-oil alkyd resin.

9. Use of an alkyd resin according to any one of the preceding claims, wherein the alkyd resin is a drying or non-drying alkyd resin.

10. Use of an alkyd resin according to any one of the preceding claims, wherein the alkyd resin further comprises a siccative.

11. Use of an alkyd resin according to any one of the preceding claims, wherein the substrate is selected from the group consisting of a polymer body, a paint, a varnish, a coating, an ink, glass fibers and/or an inorganic oxide material.

12. Use of an alkyd resin according to any one of the preceding claims, wherein the alkyd resin is further combined with another polymer, such as a thermoplastic polymer, a thermoplastic elastomer, a duroplast or an elastomer.

13. Use of an alkyd resin according to any one of the preceding claims, wherein the alkyd resin additionally reduces the growth of a biofilm on the surface of the substrate.

14. Use of an alkyd resin according to any of the preceding claims, wherein the alkyd resin does not contain any other internal or external biocidal compound, preferably no other internal biocidal compound.

Figure 1a

20μm

| EHT = 5.00 kV | Signal A = SE1 | LEO 1530 VP |
| Mag = 500 X | WD = 10 mm | User Text = |

EP 4 047 063 A1

Figure 1c

20µm    EHT = 5.00 kV    Signal A = SE1    LEO 1530 VP
        Mag = 500 X      WD = 10 mm        User Text =

EP 4 047 063 A1

Figure 1e

Setal 196 XX

Day 13

EHT = 5.00 kV
Mag = 500 X
Signal A = SE1
WD = 10 mm
LEO 1530 VP
User Text =

20µm

Figure 1f

EP 4 047 063 A1

**Figure 2a**          Alkyd/Melamine resin          Day 2

20µm          EHT = 5.00 kV          Signal A = SE1          LEO 1530 VP
                    Mag = 500 X          WD = 10 mm          User Text =

Figure 2c

Alkyd/Melamine resin

Day 21

EHT = 5.00 kV    Signal A = SE1    LEO 1530 VP
Mag = 500 X      WD = 10 mm       User Text =

20µm

**Figure 2d** Blank value (PP-plate without resin) Day 2

| 20µm | EHT = 5.00 kV | Signal A = SE1 | LEO 1530 VP |
| | Mag = 500 X | WD = 10 mm | User Text = |

Figure 2e

Blank value (PP-plate without resin)

Day 12

EHT = 5.00 kV   Signal A = SE1   LEO 1530 VP
Mag = 500 X   WD = 10 mm   User Text =

20µm

**Figure 2f**                    Blank value (PP-plate without resin)                    Day 21

20µm                    EHT = 5.00 kV              Signal A = SE1          LEO 1530 VP
                       Mag = 500 X                WD = 10 mm            User Text =

Europäisches
Patentamt

European
Patent Office

Office européen
des brevets

# EUROPEAN SEARCH REPORT

Application Number

EP 22 16 8879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 1 595 738 A (SAPOLIN PAINTS) 19 August 1981 (1981-08-19) * page 1, line 36 – page 2, line 100; claims 1-4 * | 1-14 | INV. C09D167/08 C09D5/16 C09D9/00 |
| X | WO 2010/040902 A1 (TIKKURILA OY [FI]; UPM KYMMENE CORP [FI] ET AL.) 15 April 2010 (2010-04-15) | 1-10, 12-14 | ADD. C02F1/50 A61L2/232 D21H21/36 |
| A | * page 2, line 13 – page 12, line 25; claim 8; examples 1, 4; tables 5, 6 * | 11 | |
| X,D | US 4 039 494 A (DRISKO RICHARD W) 2 August 1977 (1977-08-02) | 1,3, 5-11,13 | |
| A | * column 2, line 11 – column 2, line 65 * | 2,4,12, 14 | |
| X | EP 0 344 565 A1 (VIANOVA KUNSTHARZ AG [AT]) 6 December 1989 (1989-12-06) | 1-10 | |
| A | * claims 1, 4-7, 9; examples 1, 4 * | 11-14 | |

TECHNICAL FIELDS SEARCHED (IPC)

C09D
A61L
C02F
D21H

The present search report has been drawn up for all claims

| Place of search | Date of completion of the search | Examiner |
|---|---|---|
| The Hague | 7 July 2022 | Enescu, Cristina |

EPO FORM 1503 03.82 (P04C01)

## ANNEX TO THE EUROPEAN SEARCH REPORT
## ON EUROPEAN PATENT APPLICATION NO.

EP 22 16 8879

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2022

| Patent document cited in search report | | Publication date | Patent family member(s) | | Publication date |
|---|---|---|---|---|---|
| GB 1595738 | A | 19-08-1981 | AU | 3372878 A | 06-09-1979 |
| | | | BR | 7803203 A | 20-03-1979 |
| | | | CA | 1105191 A | 14-07-1981 |
| | | | DE | 2809934 A1 | 23-11-1978 |
| | | | FR | 2391257 A1 | 15-12-1978 |
| | | | GB | 1595738 A | 19-08-1981 |
| | | | JP | S5416539 A | 07-02-1979 |
| | | | NL | 7805423 A | 22-11-1978 |
| | | | US | 4071514 A | 31-01-1978 |
| WO 2010040902 | A1 | 15-04-2010 | DK | 2352624 T3 | 14-06-2021 |
| | | | EA | 201170538 A1 | 30-12-2011 |
| | | | EP | 2352624 A1 | 10-08-2011 |
| | | | FI | 20085953 A | 10-04-2010 |
| | | | LT | 2352624 T | 26-07-2021 |
| | | | PL | 2352624 T3 | 08-11-2021 |
| | | | UA | 105773 C2 | 25-06-2014 |
| | | | WO | 2010040902 A1 | 15-04-2010 |
| US 4039494 | A | 02-08-1977 | NONE | | |
| EP 0344565 | A1 | 06-12-1989 | AT | 390442 B | 10-05-1990 |
| | | | DK | 252389 A | 01-12-1989 |
| | | | EP | 0344565 A1 | 06-12-1989 |
| | | | HU | 208988 B | 28-02-1994 |
| | | | NO | 172106 B | 01-03-1993 |
| | | | PL | 161231 B1 | 30-06-1993 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4039494 A
- WO 201004903 A1

- WO 2010040903 A